# EUROPEAN PATENT SPECIFICATION

(11) **EP 1 058 559 B1**
(45) Date of publication and mention of the grant of the patent: **12.05.2004**
(21) Application number: 99906366.2
(22) Date of filing: 26.02.1999
(51) Int. Cl.: A61K 45/06, A61K 31/445, A61K 31/18

(54) **COMBINATION OF A SELECTIVE NMDA NR2B ANTAGONIST AND A COX-2 INHIBITOR**
KOMBINATIONSPRÄPARAT, DAS SELEKTIVE NMDA NR2B-ANTAGONISTEN UND COX-2 INHIBITOREN ENTHÄLT
COMBINAISON D'UN ANTAGONISTE SELECTIF DE NMDA NR2B ET D'UN INHIBITEUR DE COX-2

(30) Priority: 06.03.1998 GB 9804886
(43) Date of publication of application: 13.12.2000
(73) Proprietor: MERCK SHARP & DOHME LTD., Hoddesdon, Hertfordshire EN11 9BU (GB)
(72) Inventor: BOYCE, Susan, Harlow,Essex CM20 2QR (GB)
(74) Representative: Horgan, James Michael Frederic
(86) International application number: PCT/GB1999/000581
(87) International publication number: WO 1999/044640

(56) References cited:
- WO-A-96/19469
- US-A- 5 409 944
- US-A- 5 521 213

## Description

This invention relates to the treatment or prevention of pain or nociception by the administration of a combination of a selective NMDA NR2B antagonist and a COX-2 inhibitor.

Pain has been defined as the sensory experience perceived by nerve tissue distinct from sensations of touch, pressure, heat and cold. It is often described by sufferers by such terms as bright, dull, aching, pricking, cutting, burning, etc. This range of sensations, as well as the variation in perception of pain by different individuals, renders a precise definition of pain near impossible. Pain as suffering, however, is generally considered to include both the original sensation and the reaction to that sensation. Where pain is "caused" by the stimulation of nociceptive receptors and transmitted over intact neural pathways, this is termed nociceptive pain. Alternatively, pain may be caused by damage to neural structures, often manifesting itself as neural supersensitivity, and is classed as neuropathic pain.

The level of stimulation at which pain is perceived is referred to as the "pain threshold". Where the pain threshold is raised, for instance, by the administration of an analgesic drug, a greater intensity or more prolonged stimulus is required before pain is experienced. Analgesics are a class of pharmaceutical agent which, following administration to a patient in need of such treatment, relieve pain without loss of consciousness. This is in contrast to other pain-relieving drugs, for example, general anaesthetics which obtund pain by producing a hiatus in consciousness, or local anaesthetics which block transmission in peripheral nerve fibres thereby preventing pain.

NMDA (N-methyl-D-aspartate)-type glutamate receptors are believed to play a pivotal role in the transmission of excitatory signals from primary sensory neurones to the brain through the spinal cord (A. H. Dickenson (1990) Trends Pharmacol. Sci., 11, 307-309). NMDA receptors mediate Ca²⁺ influx into neurones, and its receptor-gated channel activity is blocked by Mg²⁺ in a voltage-dependant manner. Subunits of the NMDA receptors are classified into two gene families, i.e., NR1 and NR2. A variety of compounds have been designed as antagonists targeting these subunits of the NMDA receptor for the treatment of neurodegenerative disorders, as well as acute and/or chronic pain and hyperalgesia. The NR2B subunit is predominantly expressed in the hippocampus (Ishii *et al.*, (1993), J. Biol. Chem. 268, 2836-2843).

NMDA antagonists such as ketamine, dextromethorphan and CPP are known to have analgesic properties in man. However, these agents also induce unacceptable side-effects including hallucinations, dysphoria and cognitive and motor disturbances (see Kristensen *et al*., 1992, Pain, 51, 249ff; Price *et al.*, 1994, Pain, 59, 165ff and Max *et al*., Clin. Neuropharmacol., 118, 360ff). In preclinical studies, dextromethorphan has been reported to potentiate the antinociceptive effects of NSAIDS and morphine (Price *et al*., 1996, Pain, 68, 119-127; Mao *et al*., 1996, Pain, 67, 361-368). However, since dextromethorphan can induce adverse effects at analgesic doses in man, it is not clear from these studies whether such combinations would still be dogged with unwanted side-effects.

One selective NMDA NR2B antagonist CP-101,606 is known to possess anti-nociceptive activity, see Taniguchi *et al*., B. J. Pharmacol., 1997, 122, 809-812. Potent analgesic activity of this compound was shown in rat hyperalgesic and nociceptive tests at doses showing no behavioural abnormality.

There is, however, no general teaching in the art that all selective NMDA NR2B antagonists are useful as analgesics, nor that they have improved motor side-effect profile compared to NMDA/glycine antagonists. Evidence for this is, for the first time, provided herein.

Furthermore there is no suggestion in the art that selective NMDA NR2B antagonists could potentiate the effects of opioids, such as morphine, thus providing analgesia with suprisingly reduced side-effects, such as motor-impairment. Thus the safety margin for the use of opioids, such as morphine, is surprisingly improved. There is no indication in the art relating to NMDA antagonists that the property of potentiating the action of morphine could be transferred to compounds selective for the NR2B subunit.

As the present specification surprisingly demonstrates that selective NMDA NR2B antagonists possess antinociceptive effects in rat models of inflammatory and neuropathic pain with a much improved side-effect window over non-competitive NMDA antagonists (ataxic/antinociceptive), when combined with an opioid, the combination is better tolerated than expected.

Inhibitors of cyclooxygenase-2 are a sub-class of the class of drugs known as non-steroidal antiinflammatory drugs (NSAIDs). The NSAIDs are active in reducing the prostaglandin-induced pain and swelling associated with the inflammation process but are also active in affecting other prostaglandin-regulated processes not associated with the inflammation process. Thus, use of high doses of most common NSAIDs can produce severe side effects, including life threatenting ulcers, that limit their therapeutic potential. An alternative to NSAIDs is the use of corticosteroids, which have even more drastic side effects, especially when long term therapy is involved.

Previous NSAIDs have been found to prevent the production of prostaglandin by inhibiting enzymes in the human arachidonic acid/prostaglandin pathway including the enzyme cyclooxygenase (COX). The recent discovery that there are two isoforms of the COX enzyme, the first, COX-1, being involved with physiological functions and the second, COX-2, being induced in inflamed tissue, has given rise to a new approach. While conventional NSAIDs block both forms of the enzyme, the identification of the inducible COX-2 enzyme associated with inflammation has provided a viable target of inhibition which more effectively reduces inflammation and produces fewer and less drastic side effects. Many compounds which have activity as COX-2 inhibitors have been identified, and much research continues in this area.

As the use of COX-2 inhibiting compounds may give rise to side-effects there is a need to develop methods which enable the clinician to use lower doses of them thereby reducing side-effects.

The present invention accordingly provides the use of a selective NMDA NR2B antagonist and a COX-2 inhibitor for the manufacture of a medicament for the treatment or prevention of pain or nociception.

In a further aspect of the present invention, there is provided a pharmaceutical composition comprising a selective NMDA NR2B antagonist and a COX-2 inhibitor, together with at least one pharmaceutically acceptable carrier or excipient.

It will be appreciated that the selective NMDA NR2B antagonist and a COX-2 inhibitor may be present as a combined preparation for simultaneous, separate or sequential use for the treatment or prevention of pain. Such combined preparations may be, for example, in the form of a twin pack.

In a further or alternative aspect of the present invention, there is therefore provided a product comprising a selective NMDA NR2B antagonist and a COX-2 inhibitor as a combined preparation for simultaneous, separate or sequential use in the treatment or prevention of pain or nociception.

The compositions of the present invention are useful for the treatment of pain of any eitiology, including acute and chronic pain and any pain with an inflammatory component. Examples of acute pain include, in particular, post-operative pain, migraine, headache and trigeminal neuralgia. Examples of chronic pain include, in particular, pain associated with musculo-skeletal disorders such as rheumatoid arthritis, osteoarthritis, ankylosing spondylitis, sero-negative (non-rheumatoid) arthropathies, non-articular rheumatism and peri-articular disorders, and pain associated with cancer, peripheral neuropathy and post-herpetic neuralgia. Examples of pain with an inflammatory component (in addition to some of those described above) include rheumatic pain, dental pain and dysmenorrhoea.

The compositions of the present invention are especially useful for the treatment of pain where the use of a COX-2 inhibitor is generally prescribed. By the use of a combination of a selective NMDA NR2B antagonist and an opioid analgesic in accordance with the present invention, it is now possible to treat pain with a sub-maximal dose of an opioid analgesic thereby reducing the likelihood of side-effects associated with opioid analgesic usage (e.g. respiratory depression, constipation, nausea and vomiting, and tolerance and dependence and the associated problem of drug withdrawal).

A particularly preferred use for a composition of the present invention is in the treatment or prevention of post-operative pain.

Selective NMDA NR2B antagonists of use in the present invention include eliprodil (and those of EP-A-109317 and French utility certificate FR 89 04835), ifenprodil (and those of French patent FR 5733 M), Ro25-6981 (and those of EP-A-648744), compounds disclosed in WO-A-9713769 to Pharmacia and CP-101,606 (and those of EP-A-768086). A particularly favoured compound is Ro25-6981:

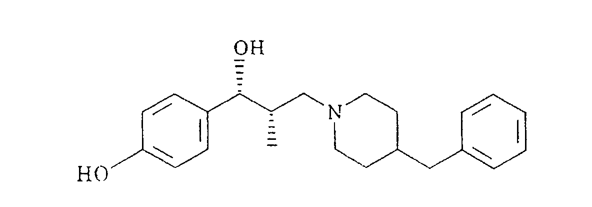

Particularly suitable selective NMDA NR2B antagonists can be identified by the following cascade which forms a further feature of the present invention. There is accordingly provided an assay for identifying a selective NMDA NR2B antagonist comprising:
(i) determining a compound having an IC₅₀ of less than 100 nM affinity at the human NMDA NR2B receptor and having a greater than 100-fold selectivity for NR2B receptors over human I_{(Kr)} cardiac potassium channels in radioligand binding studies;
(ii) demonstrating said compound inhibits hyperalgesia with ID₅₀ <30mg/kg i.p. or s.c. and has a greater than 10-fold window between doses producing antinociception and motor disruption in carrageenan-induced hyperalgesia in rats;
(iii) determining said compounds has an ID₅₀ of less than 30mg/kg i.p. or s.c. in the rat sciatic nerve ligation assay of neuropathic pain;
(iv) determining said compound has an ID₅₀ of less than 30mg/kg p.o. in the rat carrageenan-induced hyperalgesia; and
(vi) demonstrating said compound has synergistic antinociceptive effects in combination with a COX-2 inhibitor in an assay of nociception such as inhibition of hyperalgesia induced by carrageenan or Freund's adjuvant or inhibition of allodynia in neuropathic rats.

The compounds of use in this invention may have one or more asymmetric centres and can therefore exist as enantiomers and possibly as diastereoisomers. It is to be understood that the present invention relates to the use of all such isomers and mixtures thereof.

Suitable pharmaceutically acceptable salts of the selective NMDA NR2B antagonists of use in the present invention include acid addition salts which may, for example, be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid or phosphoric acid. Salts of amine groups may also comprise the quaternary ammonium salts in which the amino nitrogen atom carries an alkyl, alkenyl, alkynyl or aralkyl group. Where the compound carries an acidic group, for example a carboxylic acid group, the present invention also contemplates salts thereof, preferably non-toxic pharmaceutically acceptable salts thereof, such as the sodium, potassium and calcium salts thereof.

The terms "inhibitor of cyclooxygenase-2", "cyclooxygenase-2 inhibitor" and "COX-2 inhibitor" as used herein embrace compounds which selectively inhibit cyclooxygenase-2 over cyclooxygenase-1. Employing the human whole blood COX-1 assay and the human whole blood COX-2 assay described in C. Brideau et al, Inflamm. Res. *45*: 68-74 (1996), preferably, the compounds have a cyclooxygenase-2 IC₅₀ of less than about 2 mM in the human whole blood COX-2 assay, yet have a cyclooxygenase-1 IC₅₀ of greater than about 5 mM in the human whole blood COX-1 assay. Also preferably, the compounds have a selectivity ratio of cyclooxygenase-2 inhibition over cyclooxygenase-1 inhibition of at least 10, and more preferably of at least 40. The resulting selectivity may indicate an ability to reduce the incidence of common NSAID-induced side effects.

It is particularly preferred that the COX-2 inhibitor be rofecoxib or celecoxib.

As explained in J. Talley, Exp. Opin. Ther. Patents (1997), 7(1), pp. 55-62, three distinct structural classes of selective COX-2 inhibitor compounds have been identified. One class is the methane sulfonanilide class of inhibitors, of which NS-398, flosulide, nimesulide and L-745,337 are example members.

A second class is the tricyclic inhibitor class, which can be further divided into the sub-classes of tricyclic inhibitors with a central carbocyclic ring (examples include SC-57666, 1, and 2); those with a central monocyclic heterocyclic ring (examples include DuP 697, SC-58125, SC-58635, and 3, 4 and 5); and those with a central bicyclic heterocyclic ring (examples include 6, 7, 8, 9 and 10). Compounds 3, 4 and 5 are described in U.S. Patent No. 5,474,995.

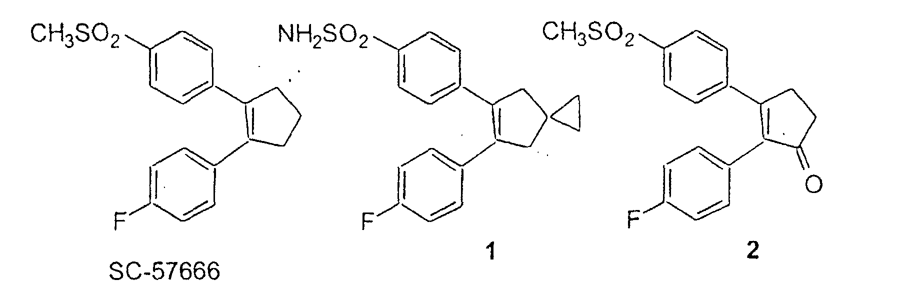

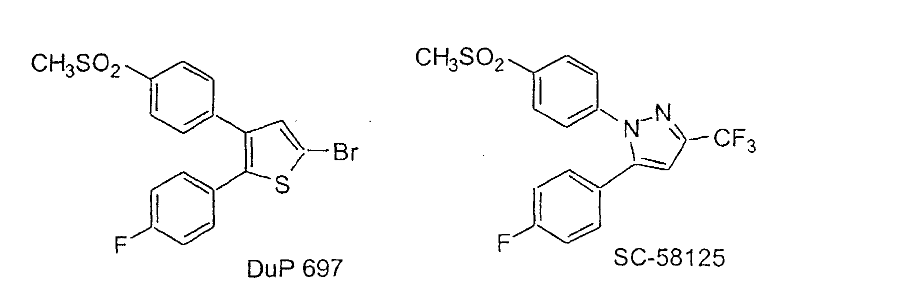

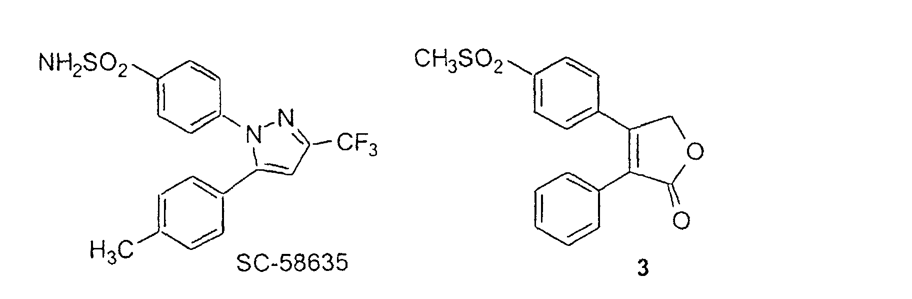

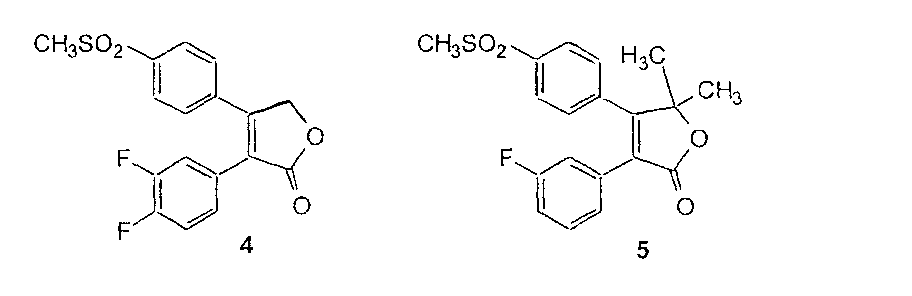

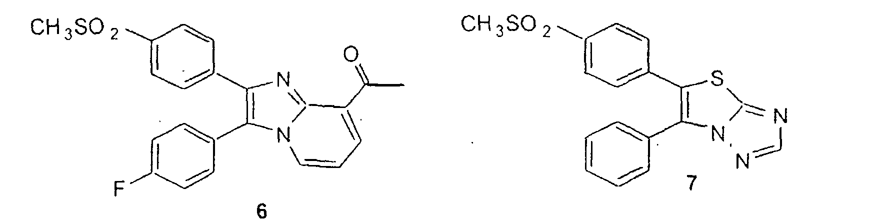

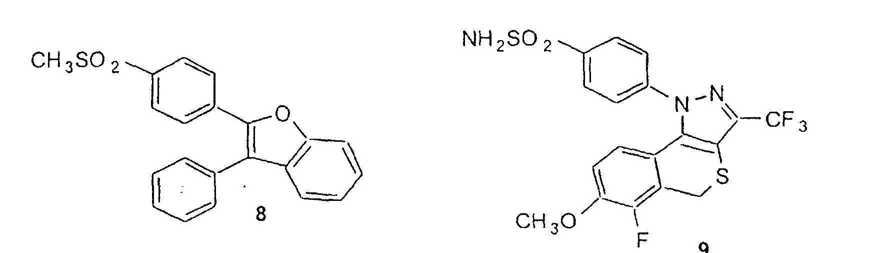

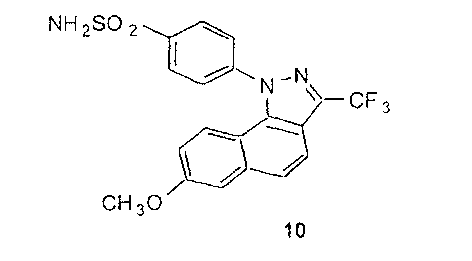

The third identified class can be referred to as those which are structurally modified NSAIDS, and includes L-761,066 and structure 11 as example members.

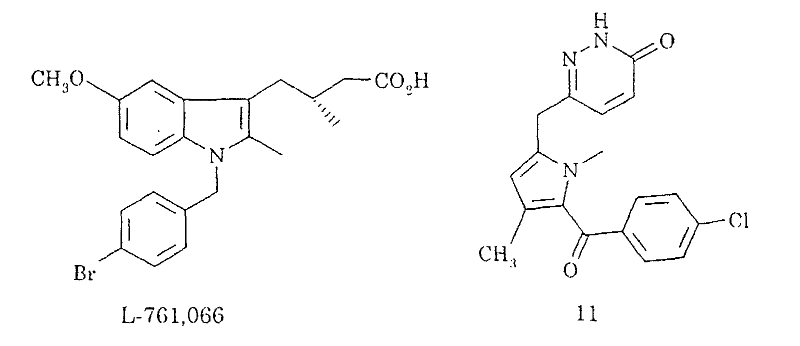

In addition to the structural classes, sub-classes, specific COX-2 inhibitor compound examples, and reference journal and patent publications described in the Talley publication which are all herein incorporated by reference, examples of compounds which selectively inhibit cyclooxygenase-2 have also been described in the following patent publications : U.S. Patent No.'s 5,344,991, 5,380,738, 5,393,790, 5,409,944, 5,434,178, 5,436,265, 5,466,823, 5,474,995, 5,510,368, 5,536,752, 5,550,142, 5,552,422, 5,604,253, 5,604,260, 5,639,780; and International Patent Specification Nos. 94/13635, 94/15932, 94/20480, 94/26731, 94/27980, 95/00501, 95/15316, 96/03387, 96/03388, 96/06840; and International Publication No.'s WO 94/20480, WO 96/21667, WO 96/31509, WO 96/36623, WO 97/14691, WO 97/16435.

Additional COX-2 inhibitor compounds which are included in the scope of this invention include:

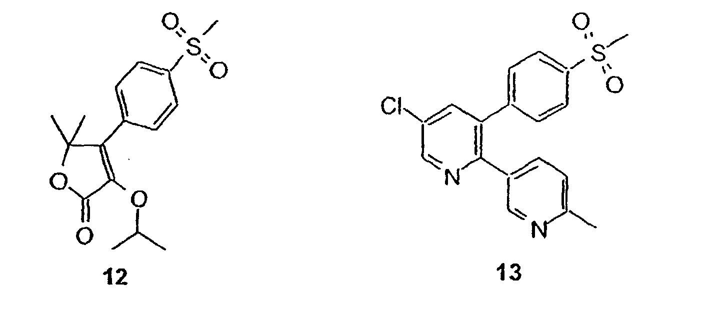

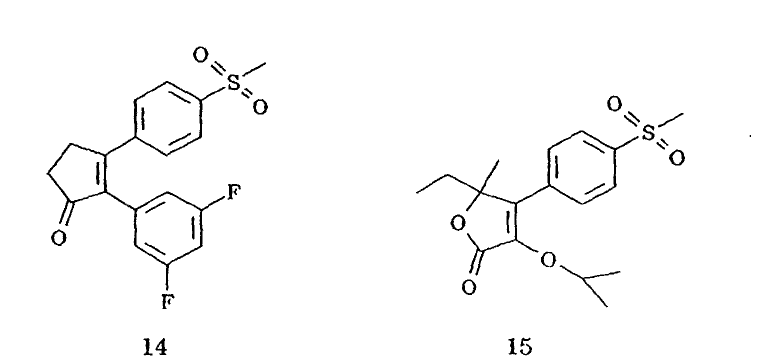

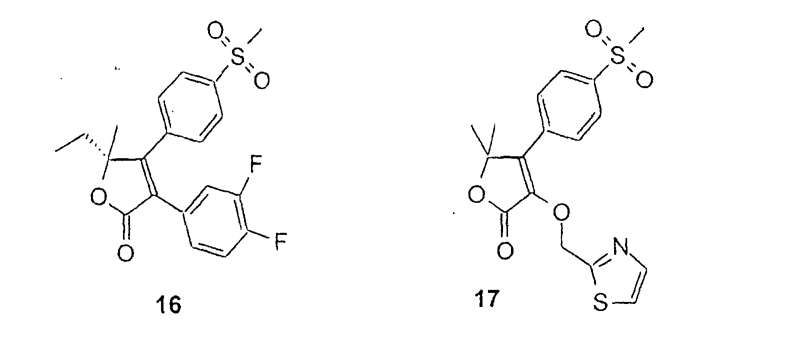

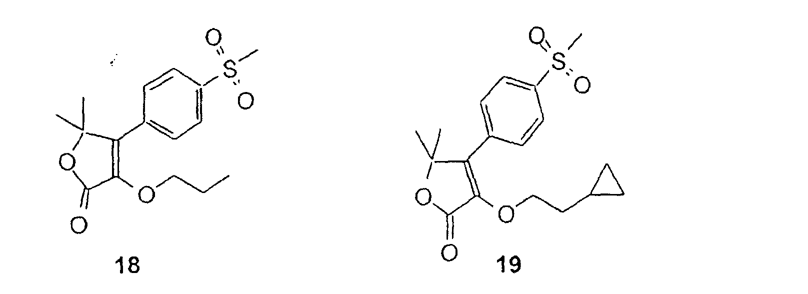

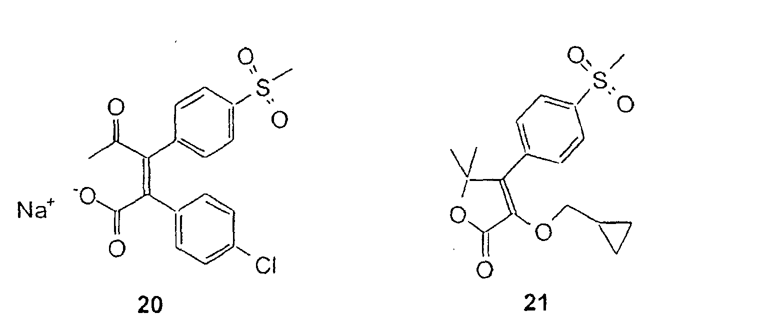

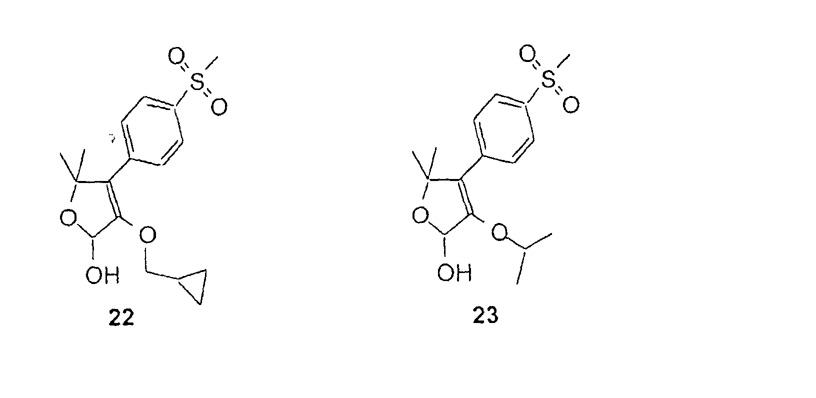

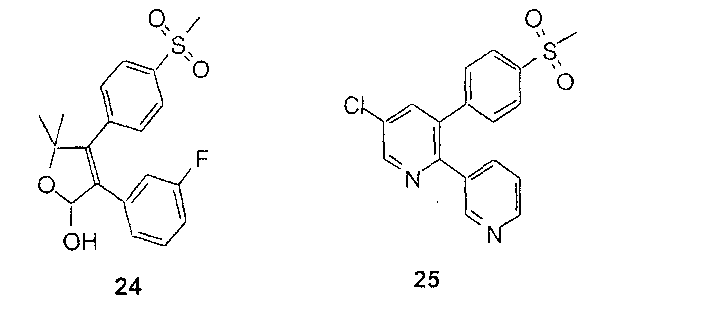

Some of the compounds above can also be identified by the following chemical names:
**3:** 3-phenyl-4-(4-(methylsulfonyl)phenyl)-2-(5H)-furanone;
**4:** 3-(3,4-difluorophenyl)-4-(4-(methylsulfonyl)phenyl)-2-(5H)-furanone;
**5:** 5,5-dimethyl-4-(4-(methylsulfonyl)phenyl)-3-(3-fluorophenyl)-5H-furan-2-one;
**12:** 5,5-dimethyl-4-(4-(methylsulfonyl)phenyl)-3-(2-propoxy)-5H-furan-2-one;
**13:** 5-chloro-3-(4-(methylsulfonyl)phenyl)-2-(2-methyl-5-pyridinyl)pyridine;
**14:** 2-(3,5-difluorophenyl)-3-(4-(methylsulfonyl)phenyl)-2-cyclopenten-1-one;
**15:** 5(S)-5-ethyl-5-methyl-4-(4-(methylsulfonyl)phenyl)-3-(2-propoxy)-5H-furan-2-one;
**16:** 5-ethyl-5-methyl-4-(4-(methylsulfonyl)phenyl)-3-(3,4-difluorophenyl)-5H-furan-2-one;
**17:** 3-((2-thiazolyl)methoxy)-4-(4-(methylsulfonyl)phenyl)-5,5-dimethyl-5H-furan-2-one;
**18:** 3-propyloxy-4-(4-(methylsulfonyl)phenyl)-5,5-dimethyl-5H-furan-2-one;
**19:** 3-(1-cyclopropylethoxy)-5,5-dimethyl-4-(4-methylsulfonyl)phenyl)-5H-furan-2-one;
**20:** sodium 2-(4-chlorophenyl)-3-(4-(methylsulfonyl)phenyl)-4-oxo-2-pentenoate;
**21:** 3-(cyclopropylmethoxy)-5,5-dimethyl-4-(4-(methylsulfonyl)phenyl)-5H-furan-2-one;
**22:** 3-(cyclopropylmethoxy)-5,5-dimethyl-4-(4-(methylsulfonyl)phenyl)-2,5-dihydrofuran-2-ol;
**23:** 3-isopropoxy-5,5-dimethyl-4-(4-(methylsulfonyl)phenyl)-2,5-dihydrofuran-2-ol;
**24:** 5,5-dimethyl-3-(3-fluorophenyl)-2-hydroxy-4-(4-(methylsulfonyl)phenyl)-2,5-dihydrofuran;
25: 5-Chloro-3-(4-(methylsulfonyl)phenyl)-2-(3-pyridinyl)pyridine.

The following publications describe and/or provide methods for making the compounds as indicated: compounds 12, 15, 17, 18, 19 and 21, WO 97/14691; compounds 22, 23 and 24, WO 97/16435; compound 20, WO 96/36623; compound 14, U.S. Patent No. 5,536,752; compound 16, U.S. Patent No. 5,474,995. See Examples herein for compounds 13 and 25.

Also incorporated herein by reference are those compounds described in WO 96/41645 as having structural Formula I, shown below, and the definition and preferred definitions and species described therein:

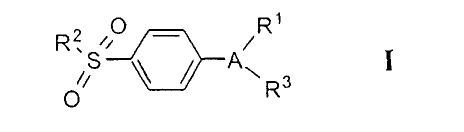

Particularly preferred compounds of formula (I) include:
5-(4-fluorophenyl)-1-[4-(methylsulfonyl)phenyl]-3-(trifluoromethyl)pyrazole;
4-(4-fluorophenyl)-5-[4-(methylsulfonyl)phenyl]-1-phenyl-3-(trifluoromethyl)pyrazole;
4-(5-(4-chlorophenyl)-3-(4-methoxyphenyl)-1H-pyrazol-1-yl)benzenesulfonamide;
4-(3,5-bis(4-methylphenyl)-1H-pyrazol-1-yl)benzenesulfonamide;
4-(5-(4-chlorophenyl)-3-phenyl-1H-pyrazol-1-yl)benzenesulfonamide;
4-(3,5-bis(4-methoxyphenyl)-1H-pyrazol-1-yl)benzenesulfonamide;
4-(5-(4-chlorophenyl)-3-(4-methylphenyl)-1H-pyrazol-1-yl)benzenesulfonamido;
4-(5-(4-chlorophenyl)-3-(4-nitrophenyl)-1H-pyrazol-1-yl)benzenesulfonamide;
4-(5-(4-chlorophenyl)-3-(5-chloro-2-thienyl)-1H-pyrazol-1-yl)benzenesulfonamide;
4-(4-chloro-3,5-diphenyl-1H-pyrazol-1-yl)benzenesulfonamide;
4-(5-(4-chlorophenyl)-3-(trifluoromethyl)-1H-pyrazol-1-yl)benzenesulfonamide;
4-(5-phenyl)-3-(trifluoromethyl)-1H-pyrazol-1-yl)benzenesulfonamide;
4-(5-(4-fluorophenyl)-3-(trifluoromethyl)-1H-pyrazol-1-yl)benzenesulfonamide;
4-(5-(4-methoxyphenyl)-3-(trifluoromethyl)-1H-pyrazol-1-yl)benzenesulfonamide;
4-(5-(4-chlorophenyl)-3-(difluoromethyl)-1H-pyrazol-1-yl)benzenesulfonamide;
4-(5-(4-methylphenyl)-3-(trifluoromethyl)-1H-pyrazol-1-yl)benzenesulfonamide;
4-(4-chloro-5-(4-chlorophenyl)-3-(trifluoromethyl)-1H-pyrazol-1-yl)benzenesulfonamide;
4-(3-(difluoromethyl)-5-(4-methylphenyl)-1H-pyrazol-1-yl)benzenesulfonamide;
4-(3-(difluoromethyl)-5-phenyl-1H-pyrazol-1-yl)benzenesulfonamide;
4-(3-(difluoromethyl)-5-(4-methoxyphenyl)-1H-pyrazol-1-yl)benzenesulfonamide;
4-(3-cyano-5-(4-fluorophenyl)-1H-pyrazol-1-yl)benzenesulfonamide;
4-(3-(difluoromethyl)-5-(3-fluoro-4-methoxyphenyl)-1H-pyrazol-1-yl)benzenesulfonamide;
4-(5-(3-fluoro-4-methoxyphenyl)-3-(trifluoromethyl)-1H-pyrazol-1-yl)benzenesulfonamide;
4-(4-chloro-5-phenyl-1H-pyrazol-1-yl)benzenesulfonamide;
4-(5-(4-chlorophenyl)-3-(hydroxyphenyl)-1H-pyraxol-1-yl)benzenesulfonamide;
4-(5-(4-(N,N-dimethylamino)phenyl)-3-(trifluoromethyl)-1H-pyrazol-1-yl)benzenesulfonamide;
5-(4-fluorophenyl)-6-(4-(methylsulfonyl)phenyl)spiro[2.4]hept-5-ene;
4-(6-(4-fluorophenyl)spiro[2.4]hept-5-en-5-yl)benzenesulfonamide;
6-(4-fluorophenyl)-7-(4-(methylsulfonyl)phenyl)spiro[3.4]oct-6-ene;
5-(3-chloro-4-methoxyphenyl)-6-(4-(methylsulfonyl)phenyl)spiro[2.4]hept-5-ene;
4-(6-(3-chloro-4-methoxyphenyl)spiro[2.4]hept-5-en-5-yl)benzenesulfonamide;
5-(3,5-dichloro-4-methoxyphenyl)-6-(4-(methylsulfonyl)phenyl)spiro[2.4]hept-5-ene;
5-(3-chloro-4-fluorophenyl)-6-(4-(methylsulfonyl)phenyl)spiro[2.4]hept-5-ene;
4-(6-(3,4-dichlorophenyl)spiro[2.4]hept-5-en-5-yl)benzenesulfonamide;
2-(3-chloro-4-fluorophenyl)-4-(4-fluorophenyl)-5-(4-methylsulfonylphenyl)thiazole;
2-(2-chlorophenyl)-4-(4-fluorophenyl)-5-(4-methylsulfonylphenyl)thiazole;
5-(4-fluorophenyl)-4-(4-methylsulfonylphenyl)-2-methylthiazole;
4-(4-fluorophenyl)-5-(4-methylsulfonylphenyl)-2-trifluoromethylthiazole;
4-(4-fluorophenyl)-5-(4-methylsulfonylphenyl)-2-(2-thienyl)thiazole;
4-(4-fluorophenyl)-5-(4-methylsulfonylphenyl)-2-benzylaminothiazole;
4-(4-fluorophenyl)-5-(4-methylsulfonylphenyl)-2-(1-propylamino)thiazole;
2-((3,5-dichlorophenoxy)methyl)-4-(4-fluorophenyl)-5-(4-(methylsulfonyl)phenyl)thiazole;
5-(4-fluorophenyl)-4-(4-methylsulfonylphenyl)-2-trifluoromethylthiazole;
1-methylsulfonyl-4-(1,1-dimethyl-4-(4-fluorophenyl)cyclopenta-2,4-dien-3-yl)benzene;
4-(4-(4-fluorophenyl)-1,1-dimethylcyclopenta-2,4-dien-3-yl)benzenesulfonamide;
5-(4-fluorophenyl)-6-(4-(methylsulfonyl)phenyl)spiro[2.4]hepta-4,6-diene;
4-(6-(4-fluorophenyl)spiro[2.4]hepta-4,6-dien-5-yl)benzenesulfonamide;
6-(4-fluorophenyl)-2-methoxy-5-(4-(methylsulfonyl)phenyl)-pyridine-3-carbonitrile;
2-bromo-6-(4-fluorop henyl)-5-(4-(methylsulfonyl)phenyl)-pyridine-3-carbonitrile;
6-(4-fluorophenyl)-5-(4-(methylsulfonyl)phenyl)-2-phenyl-pyridine-3-carbonitrile;
4-(2-(4-methylpyridin-2-yl)-4-(trifluoromethyl)-1H-imidazol-1-yl)benzenesulfonamide;
4-(2-(5-methylpyridin-3-yl)-4-(trifluoromethyl)-1H-imidazol-1-yl)benzenesulfonamide;
4-(2-(2-methylpyridin-3-yl)-4-(trifluoromethyl)-1H-imidazol-1-yl)benzenesulfonamide;
3-(1-(4-(methylsulfonyl)phenyl)-4-(trifluoromethyl)-1H-imidazol-2-yl)benzenesulfonamide;
2-(1-(4-(methylsulfonyl)phenyl)-4-(trifluoromethyl)-1H-imidazol-2-yl)pyridine;
2-methyl-4-(1-(4-(methylsulfonyl)phenyl)-4-(trifluoromethyl)-1H-imidazol-2-yl)pyridine;
2-methyl-6-(1-(4-(methylsulfonyl)phenyl)-4-(trifluoromethyl)-1H-imidazol-2-yl)pyridine;
4-(2-(6-methylpyridin-3-yl)-4-(trifluoromethyl)-1H-imidazol-1-yl)benzenesulfonamide;
2-(3,4-difluorophenyl)-1-(4-(methylsulfonyl)phenyl)-4-(trifluoromethyl)-1H-imidazole;
4-(2-(4-methylphenyl)-4-(trifluoromethyl)-1H-imidazol-1-yl)benzenesulfonamide;
2-(4-chlorophenyl)-1-(4-(methylsulfonyl)phenyl)-4-methyl-1H-imidazole;
2-(4-chlorophenyl)-1-(4-(methylsulfonyl)phenyl)-4-phenyl-1H-imidazole;
2-(4-chlorophenyl)-4-(4-fluorophenyl)-1-(4-(methylsulfonyl)phenyl)-1H-imidazole;
2-(3-fluoro-4-methoxyphenyl)-1-(4-(methylsulfonyl)phenyl)-4-(trifluoromethyl)-1H-imidazole;
1-(4-(methylsulfonyl)phenyl)-2-phenyl-4-trifluoromethyl-1H-imidazole;
2-(4-methylphenyl)-1-(4-(methylsulfonyl)phenyl)-4-trifluoromethyl-1H-imidazole;
4-(2-(3-chloro-4-methylphenyl)-4-(trifluoromethyl)-1H-imidazol-1-yl)benzenesulfonamide;
2-(3-fluoro-5-methylphenyl)-1-(4-(methylsulfonyl)phenyl)-4-(trifluoromethyl)-1H-imidazole;
4-(2-(3-fluoro-5-methylphenyl)-4-(trifluoromethyl)-1H-imidazol-1-yl)benzenesulfonamide;
2-(3-methylphenyl)-1-(4-(methylsulfonyl)phenyl)-4-(trifluoromethyl)-1H-imidazole;
4-(2-(3-methylphenyl)-4-(trifluoromethyl)-1H-imidazol-1-yl)benzenesulfonamide;
1-(4-(methylsulfonyl)phenyl)-2-(3-chlorophenyl)-4-(trifluoromethyl)-1H-imidazole;
4-(2-(3-chlorophenyl)-4-(trifluoromethyl)-1H-imidazol-1-yl)benzenesulfonamide;
4-(2-phenyl-4-(trifluoromethyl)-1H-imidazol-1-yl)benzenesulfonamide;
4-(2-(4-methoxy-3-chlorophenyl)-4-(trifluoromethyl)-1H-imidazol-1-yl)benzenesulfonamide;
1-allyl-4-(4-fluorophenyl)-3-(4-(methylsulfonyl)phenyl)-5-(trifluoromethyl)-1H-pyrazole;
4-(1-ethyl-4-(4-fluorophenyl)-5-(trifluoromethyl)-1H-pyrazol-3-yl)benzenesulfonamide;
N-phenyl-(4-(4-fluorophenyl)-3-(4-(methylsulfonyl)phenyl)-5-(trifluoromethyl)-1H-pyrazol-1-yl)acetamide;
ethyl (4-(4-fluorophenyl)-3-(4-(methylsulfonyl)phenyl)-5-(trifluoromethyl)-1H-pyrazol-1-yl)acetate;
4-(4-fluorophenyl)-3-(4-(methylsulfonyl)phenyl)-1-(2-phenylethyl)-1H-pyrazole;
4-(4-fluorophenyl)-3-(4-(methylsulfonyl)phenyl)-1-(2-phenylethyl)-5-(trifluoromethyl)pyrazole;
1-ethyl-4-(4-fluorophenyl)-3-(4-(methylsulfonyl)phenyl)-5-(trifluoromethyl)-1H-pyrazole;
5-(4-fluorophenyl)-4-(4-(methylsulfonyl)phenyl)-2-(trifluoromethyl)-1H-imidazole;
4-(4-(methylsulfonyl)phenyl)-5-(2-thiophenyl)-2-(trifluoromethyl)-1H-imidazole;
5-(4-fluorophenyl)-2-methoxy-4-(4-(methylsulfonyl)phenyl)-6-(trifluoromethyl)pyridine;
2-ethoxy-5-(4-fluorophenyl)-4-(4-(methylsulfonyl)phenyl)-6-(trifluoromethyl)pyridine;
5-(4-fluorophenyl)-4-(4-(methylsulfonyl)phenyl)-2-(2-propynyloxy)-6-(trifluoromethyl)pyridine;
2-bromo-5-(4-fluorophenyl)-4-(4-(methylsulfonyl)phenyl)-6-(trifluoromethyl)pyridine;
4-(2-(3-chloro-4-methoxyphenyl)-4,5-difluorophenyl)benzenesulfonamide;
1-(4-fluorophenyl)-2-(4-(methylsulfonyl)phenyl)benzene;
5-difluoromethyl-4-(4-(methylsulfonyl)phenyl)-3-phenylisoxazole;
4-(3-ethyl-5-phenylisoxazol-4-yl)benzenesulfonamide;
4-(5-difluoromethyl-3-phenylisoxazol-4-yl)benzenesulfonamide;
4-(5-hydroxymethyl-3-phenylisoxazol-4-yl)benzenesulfonamide;
4-(5-methyl-3-phenylisoxazol-4-yl)benrenesulfonamide;
1-(2-(4-fluorophenyl)cyclopenten-1-yl)-4-(methylsulfonyl)benzene;
1-(2-(4-fluoro-2-methylphenyl)cyclopenten-1-yl)-4-(methylsulfonyl)benzene;
1-(2-(4-chlorophenyl)cyclopenten-1-yl)-4-(methylsulfonyl)benzene;
1-(2-(2,4-dichlorophenyl)cyclopenten-1-yl)-4-(methylsulfonyl)benzene;
1-(2-(4-trifluoromethylphenyl)cyclopenten-1-yl)-4-(methylsulfonyl)benzene;
1-(2-(4-methylthiophenyl)cyclopenten-1-yl)-4-(methylsulfonyl)benzene;
1-(2-(4-fluorophenyl)-4,4-dimethylcyclopenten-1-yl)-4-(methylsulfonyl)benzene;
4-(2-(4-fluorophenyl)-4,4-dimethylcyclopenten-1-yl)benzenesulfonamide;
1-(2-(4-chlorophenyl)-4,4-dimethylcyclopenten-1-yl)-4-(methylsulfonyl)benzene;
4-(2-(4-chlorophenyl)-4,4-dimethylcyclopenten-1-yl)benzenesulfonamide;
4-(2-(4-fluorophenyl)cyclopenten-1-yl)benzenesulfonamide;
4-(2-(4-chlorophenyl)cyclopenten-1-yl)benzenesulfonamide;
1-(2-(4-methoxyphenyl)cyclopenten-1-yl)-4-(methylsulfonyl)benzene;
1-(2-(2,3-difluorophenyl)cyclopenten-1-yl)-4-(methylsulfonyl)benzene;
4-(2-(3-fluoro-4-methoxyphenyl)cyclopenten-1-yl)benzenesulfonamide;
1-(2-(3-chloro-4-methoxyphenyl)cyclopenten-1-yl)-4-(methylsulfonyl)benzene;
4-(2-(3-chloro-4-fluorophenyl)cyclopenten-1-yl)benzenesulfonamide;
4-(2-(2-methylpyridin-5-yl)cyclopenten-1-yl)benzenesulfonamide;
ethyl 2-(4-(4-fluorophenyl)-5-(4-(methylsulfonyl)phenyl)oxazol-2-yl)-2-benzyl-acetate;
2-(4-(4-fluorophenyl)-5-(4-(methylsulfonyl)phenyl)oxazol-2-yl)acetic acid;
2-(tert-butyl)-4-(4-fluorophenyl)-5-(4-(methylsulfonyl)phenyl)oxazole;
4-(4-fluorophenyl)-5-(4-(methylsulfonyl)phenyl)-2-phenyloxazole;
4-(4-fluorophenyl)-2-methyl-5-(4-(methylsulfonyl)phenyl)oxazole; and
4-(5-(3-fluoro-4-methoxyphenyl)-2-trifluoromethyl-4-oxazolyl)benzenesulfonamide;
or a pharmaceutically acceptable salt thereof.

The compounds of use in this invention may have one or more chiral centers and the present compounds may occur as racemates, racemic mixtures and as individual diasteriomers or enantiomers with all such isomeric forms and mixtures thereof being included within the scope of this invention. Furthermore, some of the crystalline forms for compounds of the present invention may exist as polymorphs and as such are intended to be included in the present invention. In addition, some of the compounds of the instant invention may form solvates with water or common organic solvents. Such solvates and hydrates, as well as anhydrous compositions, are encompassed within the scope of this invention. Some of the compounds described herein may contain olefinic double bonds, and unless specified otherwise, are meant to include both E and Z geometric isomers.

The COX-2 inhibitors that may be used with this invention encompass all pharmaceutically acceptable salt forms of the compounds. Examples of such salt forms of COX-2 inhibitors include but are not limited to salts derived from inorganic bases including aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

As stated above, the selective NMDA NR2B antagonist and COX-2 inhibitor may be formulated in a single pharmaceutical composition or alternatively in individual pharmaceutical compositions for simultaneous, separate or sequential use in accordance with the present invention.

Preferably the compositions according to the present invention are in unit dosage forms such as tablets, pills, capsules, powders, granules, solutions or suspensions, or suppositories, for oral, parenteral or rectal administration, or administration by inhalation or insufflation.

For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules.
This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

Preferred compositions for administration by injection include those comprising a selective NMDA NR2B antagonist, as the active ingredient, in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (e.g. Tween™ 20, 40, 60, 80 or 85) and other sorbitans (e.g. Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn™, Infonutrol™, Lipofundin™ and Lipiphysan™. The active ingredient may be either dissolved in a premixed emulsion composition or alternatively it may be dissolved in an oil (e.g. soybean oil, safflower oil, cottonseed oil, sesame oil, corn oil or almond oil) and an emulsion formed upon mixing with a phospholipid (e.g. egg phospholipids, soybean phospholipids or soybean lecithin) and water. It will be appreciated that other ingredients may be added, for example gylcerol or glucose, to adjust the tonicity of the emulsion. Suitable emulsions will typically contain up to 20% oil, for example, between 5 and 20%. The fat emulsion will preferably comprise fat droplets between 0.1 and 1.0µm, particularly 0.1 and 0.5µm, and have a pH in the range of 5.5 to 8.0.

Particularly preferred emulsion compositions are those prepared by mixing a selective NMDA NR2B antagonist with Intralipid™ or the components thereof (soybean oil, egg phospholipids, glycerol and water).

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising device may be attached to a face mask, tent or intermittent positive pressure breathing machine. Solution, suspension or powder compositions may be administered, preferably orally or nasally, from devices which deliver the formulation in an appropriate manner.

The present invention futher provides a process for the preparation of a pharmaceutical composition comprising a selective NMDA NR2B antagonist and a COX-2 inhibitor, which process comprises bringing a selective NMDA NR2B antagonist and a COX-2 inhibitor into association with a pharmaceutically acceptable carrier or excipient.

When administered in combination, either as a single or as separate pharmaceutical composition(s), the selective NMDA NR2B antagonist and the COX-2 inhibitor are presented in a ratio which is consistent with the manifestation of the desired effect. In particular, the ratio by weight of the selective NMDA NR2B antagonist to the COX-2 inhibitor will suitably be approximately 1 to 1. Preferably this ratio will be between 0.001 to 1 and 1000 to 1, and especially between 0.01 to 1 and 100 to 1.

A suitable dosage level for the selective NMDA NR2B antagonist is about 0.001 to 25mg/kg per day, preferably about 0.005 to 10mg/kg per day, and especially about 0.005 to 5mg/kg per day. The compounds may be administered on a regimen of up to 6 times per day, preferably 1 to 4 times per day.

The COX-2 inhibitor may be administered at a dosage level up to conventional dosage levels for such analgesics, but preferably at a reduced level in accordance with the present invention. Suitable dosage levels will depend upon the analgesic effect of the chosen COX-2 inhibitor, but typically suitable levels will be about 0.001 to 25mg/kg per day, preferably 0.005 to 10mg/kg per day, and especially 0.005 to 5mg/kg per day. The compound may be administered on a regimen of up to 6 times per day, preferably 1 to 4 times per day.

It will be appreciated that the amount of a selective NMDA NR2B antagonist and COX-2 inhibitor required for use in the treatment or prevention of pain or nociception will vary not only with the particular compounds or compositions selected but also with the route of administration, the nature of the condition being treated, and the age and condition of the patient, and will ultimately be at the discretion of the attendant physician.
The following examples illustrate pharmaceutical compositions according to the invention.

These formulations may be prepared with separate active ingredients or with with a combination of active ingredients in one composition. In such combined preparations, the ratio of selective NMDA NR2B antagonist to COX-2 inhibitor will depend upon the choice of active ingredients.

### EXAMPLE 1A Tablets containing 1-25mg of compound

| | Amount mg | | |
|---|---|---|---|
| Active Ingredients(s) | 1.0 | 2.0 | 25.0 |
| Microcrystalline cellulose | 20.0 | 20.0 | 20.0 |
| Modified food corn starch | 20.0 | 20.0 | 20.0 |
| Lactose | 58.5 | 57.5 | 34.5 |
| Magnesium Stearate | 0.5 | 0.5 | 0.5 |

### EXAMPLE 1B Tablets containing 26-100mg of compound

| | Amount mg | | |
|---|---|---|---|
| Active Ingredients(s) | 26.0 | 50.0 | 100.0 |
| Microcrystalline cellulose | 80.0 | 80.0 | 80.0 |
| Modified food corn starch | 80.0 | 80.0 | 80.0 |
| Lactose | 213.5 | 189.5 | 139.5 |
| Magnesium Stearate | 0.5 | 0.5 | 0.5 |

The active ingredient(s) cellulose, lactose and a portion of the corn starch are mixed and granulated with 10% corn starch paste. The resulting granulation is sieved, dried and blended with the remainder of the corn starch and the magnesium stearate. The resulting granulation is then compressed into tablets containing 1.0mg, 2.0mg, 25.0mg, 26.0mg, 50.0mg and 100mg of the active compound per tablet.

### EXAMPLE 2 Parenteral injection

| | Amount |
|---|---|
| Active Ingredient(s) | 1 to 100mg |
| Citric Acid Monohydrate | 0.75mg |
| Sodium Phosphate | 4.5mg |
| Sodium Chloride | 9mg |
| Water for injection | to 10ml |

The sodium phosphate, citric acid monohydrate and sodium chloride are dissolved in a portion of the water. The active ingredient(s) is (are) dissolved or suspended in the solution and made up to volume.

### EXAMPLE 3 Topical formulation

| | Amount |
|---|---|
| Active Ingredient(s) | 1-10g |
| Emulsifying Wax | 30g |
| Liquid paraffin | 20g |
| White Soft Paraffin | to 100g |

The white soft paraffin is heated until molten. The liquid paraffin and emulsifying wax are incorporated and stirred until dissolved. The active ingredient(s) is (are) is added and stirring continued until dispersed. The mixture is then cooled until solid.

### Example 4A - (Surface-Active Agent) Injection Formulation

| | |
|---|---|
| Active Ingredient(s) | up to 10mg/kg |
| Tween 80™ | up to 2.5% |
| [in 5% aqueous mannitol (isotonic)] | |

The active ingredient(s) is (are) dissolved directly in a solution of the commercially available Tween 80™ (polyoxyethylenesorbitan monooleate) and 5% aqueous mannitol (isotonic).

### Example 4B - (Emulsion) Injection Formulation

| | |
|---|---|
| Active Ingredient(s) | up to 30mg/ml |
| Intralipid™ (10-20%) | |

The active ingredient(s) is (are) dissolved directly in the commercially available Intralipid™ (10 or 20%) to form an emulsion.

### Example 4C - Alternative (Emulsion) Injectable Formulation

| | Amount |
|---|---|
| Active Ingredient(s) | 0.1 - 10mg |
| Soybean oil | 100mg |
| Egg Phospholipid | 6mg |
| Glycerol | 22mg |
| Water for injection | to 1ml |

All materials are sterilized and pyrogen free. The active ingredient(s) is (are) dissolved in soybean oil. An emulsion is then formed by mixing this solution with the egg phospholipid, glycerol and water. The emulsion is then sealed in sterile vials.

The following Example illustrates that selective NMDA NR2B receptor antagonists have a reduced motor side-effect profile when compared with NMDA/glycine antagonists.

### EXAMPLE 5

The present Example examined whether NMDA NR2B receptor antagonists have an improved therapeutic window over unselective NNIDA/glycine antagonists and other ion channel blockers including lamotrigine and gabapentin, by comparing their anti-algesic effects with their liability to induce motor impairment in rats. Anti-algesic activity was assessed using an assay of neuropathic pain in rats (sciatic nerve ligation) and in a carrageenan-induced hyperalgesia assay. Motor impairment was measured using an accelerating rotarod.

### Methods

For sciatic nerve ligation, male Sprague Dawley rats (180-220g) were anaesthetised with isofluorane, the left sciatic nerve exposed and 4 chromic catgut (4.0) ligatures were tied loosely around the nerve (spaced 1-2 mm apart) immediately proximal to the point of trifurcation. In shamoperated animals, the same dissection was performed but without ligation. Responses to mechanical pressure were assessed 7 days after ligation using a modified Randall-Selitto algesiometer in which constant force of 40 mmHg was applied to the hind paw and the latency to struggle was recorded as the reaction time. Mechanical allodynia was defined as the difference in reaction time for sham and ligature rats. Reaction times for drug treated rats were expressed as a percentage of this response. Compounds were administered 1 h before the test.
In the carrageenan-induced hyperalgesia assay, male Sprague Dawley rats (100-120 g) received an intraplantar injection of carrageenan (4.5 mg) and mechanical thresholds were determined 3 h later using a modified Ugo Basile Algesiometer. Control rats received saline (0.15 ml i.pl.). Hyperalgesia was defined as the difference in vocalisation threshold for saline- and carrageenan-injected rats. Paw pressure scores for drug-treated rats were expressed as a percentage of this response. Compounds were administered 2 h after carrageenan.
To determine the effects of the compounds on motor co-ordination, male Sprague Dawley rats (160-180 g) were first trained to remain for 120 s on the rotarod apparatus revolving at 12 r.p.m. on the morning before the test. Animals then received drug treatments and 1 h later were placed on an accelerating rotarod (increasing from 4 - 40 r.p.m. during a 5 min period) and the time the rats were able to remain on the rotarod recorded. Lamotrigine, gabapentin and (±)-CP-101,606 were suspended in 0.5% methocel and administered orally (1 ml/kg). Ifenprodil, L-701,324, L-687,414 and (±)-Ro25-6981 were dissolved or suspended in in 0.5% methocel and administered intraperitoneally (1 ml/kg). MK-801 was dissolved in distilled water and given i.p. (1 ml/kg). Doses of compounds refer to the free base.

### Results

### Anti-algesia studies

Animals with sciatic nerve ligation exhibited mechanical allodynia as measured by the reduction in the reaction time to withdraw the injured limb from the paw pressure apparatus Reaction times for sham and ligature rats were typically 22 ± 1 s and 8 ± 1 s, respectively. The NMDA NR2B antagonists, (±)-CP-101,606 and (±)-Ro25-6981, the NMDA/glycine receptor antagonist L-701,324 and partial agonist L-687,414 and the non-competitive NMDA antagonist MK-801 dose-dependently reversed mechanical allodynia induced by sciatic nerve ligation (Table 1). Similarly, the novel anti-convulsant drugs, lamotrigine and gabapentin, and the vasodilator ifenprodil, which has affinity for the NMDA NR2B receptor, reversed mchanical allodynia. The order of potency was: MK-801 > L-701,324 > (±)-Ro25-6981 > (±)-CP-101,606 > ifenprodil > L-687,414 > lamotrigine > gabapentin.

Intraplantar injection of carrageenan (4.5 mg) induced marked paw oedema and hyperalgesia to mechanical compression of the inflamed hind paw. All the compounds caused a dose-dependent inhibition of mechnical hyperalgesia induced by carrageenan (Table 1). The order of potency was: MK-801 > L-701,324 > (±)-Ro25-6981 > ifenprodil > (±)-CP-101,606 > L-687,414 = gabapentin > lamotrigine.

### Effects on behaviour and motor co-ordination

Vehicle-treated rats were able to remain on the accelerating rotarod for approximately 140 s. MK-801 dose-dependently induced impairments in rotarod performance with an ID₅₀ of 0.22 mg/kg i.p. Body rolling, and head weaving were also observed following 0.3 and 1 mg/kg doses of MK-801. The NMDA/glycine receptor antagonist L-701,324 and partial agonist L-687,414 also induced rotarod deficits (ID₅₀ of 1.9 mg/kg i.p. and 53.3 mg/kg i.p.); ataxia was evident at 10 and 30 mg/kg i.p. of L-701,324 and L-687,414 induced body rolling and ataxia at 100 and 300 mg/kg i.p. Ifenprodil caused approximately 50% impairment in rotarod performance at 50 mg/kg, however, severe adverse effects were also observed at this dose (ptosis, pilorecetion, hypoactivity and hyperventilation). Gabapentin caused motor impairments (ataxia) at doses of 30-300 mg/kg (ID₅₀ for rotarod was 133 mg/kg i.p.). Administration of lamotrigine at 500 mg/kg p.o. caused a 40% inhibition in the time spent on the rotarod; no other effects were observed at this dose. Similarly, (±)-Ro25-6981 induced a 47% inhibition of rotarod performance at a dose of 100 mg/kg i.p. (±)-CP-101,606 did not inhibit motor performace on the rotarod up to 300 mg/kg p.o.; in fact, there was a significant increase in latency following 300 mg/kg dose compared to vehicle-treated rats.

The mean ratios for the ID₅₀ inducing motor impairment and inhibition of allodynia in neuropathic rats were at least 4 fold greater for the NMDA NR2B receptor antagonists, (±)-CP-101,606 (ratio >49) and (±)-Ro25-6981 (ratio ≥26), than were found for MK-801 (ratio 1.1), the NMDA/glycine antagonists (ratio <6), gabapentin (ratio 1.5) and ifenprodil (ratio 5.3). Lamotrigine had a similar profile to that of the NMDA NR2B receptor antagonists (ratio >45).

### Conclusions

These data suggest that NMDA NR2B antagonists may he useful for treating neuropathic pain in man with an improved therapeutic window over clinically used unselective ion channel blockers.

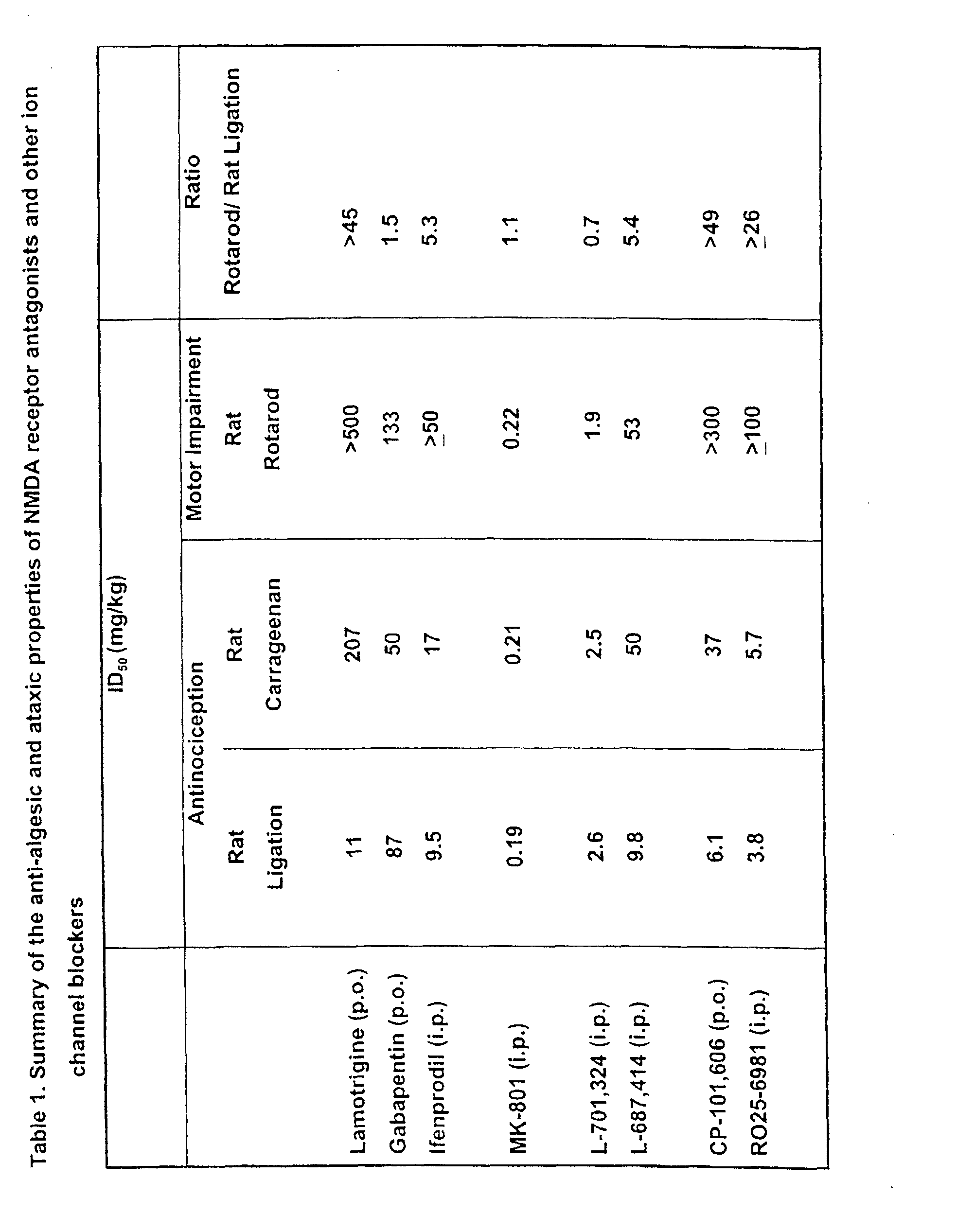

The following Example demonstrates the synergistic antinociceptive effects of a combined treatment of the selective NMDA NR2B antagonist (±)-Ro2o-6981 with the COX-2 inhibitor L-745,337:

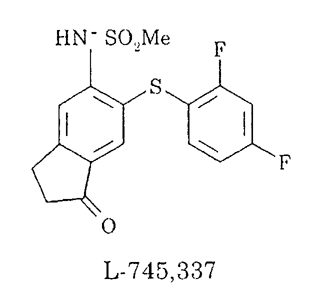

### EXAMPLE 6

The present Example examined whether the NMDA NR2B antagonist (±)-Ro25-6981 could potentiate the antinociceptive effects of a COX-2 inhibitor, L-745,337, in an assay of inflammatory hyperalgesia in rats to determine whether such combination therapies may provide improved analgesic efficacy in man with reduced side-effects.

### Methods

Mate Sprague Dawley rats (100-120 g) received an intraplantar injection of carrageenan (4.5 mg) and mechanical thresholds were determined 3 h later using a modified Ugo Basile Algesiometer. Control rats received saline (0.15 ml i.pl.). Hyperalgesia was defined as the difference in vocalisation threshold for saline- and carrageenan-injected rats. Paw pressure scores for drug-treated rats were expressed as a percentage of this response. Compounds were administered 2 h after carrageenan.

To determine the effects of morphine and (±)-Ro25-6981 on motor co-ordination, male Sprague Dawley rats (160-180 g) were first trained to remain for 120 s on the rotarod apparatus revolving at. 12 r.p.m. on the morning before the test. Animals then received drug treatments and 1 h later were placed on an accelerating rotarod (increasing from 4 - 40 r.p.m. during a 5 min period) and the time the rats were able to remain on the rotarod recorded.

(±)-Ro25-6981 was suspended in 0.5% methocel and administered intraperitoneally (1 ml/kg). L-745,337 was suspended in 0.5% methocel and given orally (2 ml/kg). Doses of compounds refer to the free base.

### Results

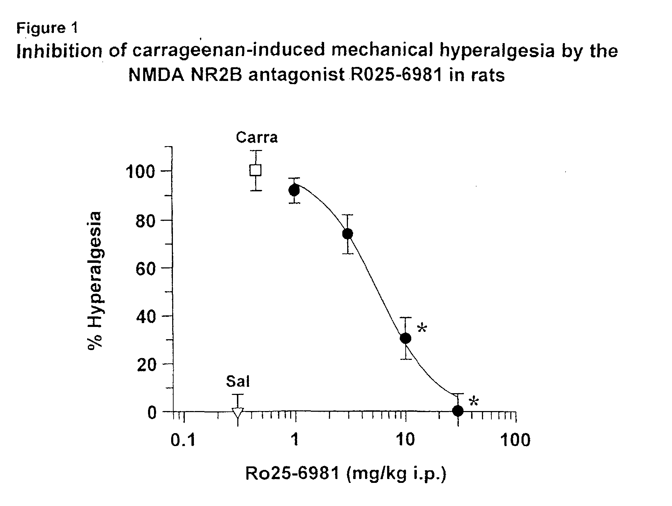

Effect of (±)-Ro25-6981 on Carrageenan-Induced Hyperalgesia Intraplantar injection of carrageenan (4.5 mg) induced marked paw oedema and hyperalgesia to mechanical compression of the inflamed hind paw. Intraperitoneal (i.p.) administration (±)-Ro25-6981 caused a dose-dependent reversal of mechanical hyperalgesia induced by carrageenan at doses of 10 and 30 mg/kg (Figure 1). A dose of 1 mg/kg was chosen for combination experiments as this did not cause significant antinociception.

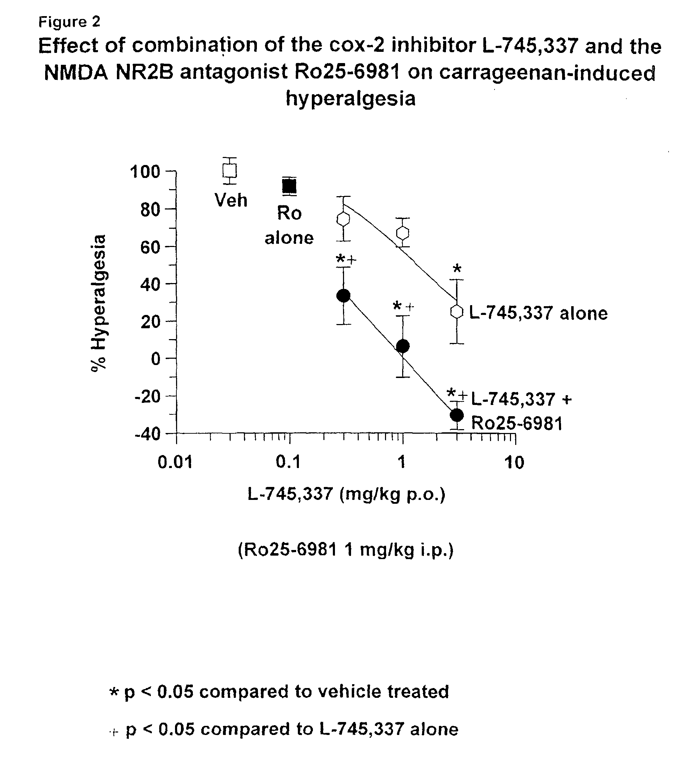

Combination of (±)-Ro25-6981 and COX-2 inhibitor L-745,337 Oral (p.o.) administration of L-745,337 (0.3-3 mg/kg) alone caused a dose-dependent inhibition of hyperalgesia which was significant at the 3 mg/kg dose alone (75% inhibition) (Figure 2). Combined treatment with (±)-Ro25-6981 (1 mg/kg i.p.) and L-745,337 (0.3-3 mg/kg) again resulted in a greater inhibition of hyperalgesia (Figure 2).

### Conclusions

These data demonstrate that administration of (±)-Ro25-6981, at a dose that is ineffective by itself, can markedly potentiate the antinociceptive effects of L-745,337. These findings suggest that NMDA NR2B receptor antagonists may be useful as combination therapy with NSAIDs to increase analgesic efficacy and reduce the incidence of side effects in man by enabling a reduction in the dose of these agents.

## Claims

1. A product comprising a selective NMDA NR2B antagonist and a COX-2 inhibitor as a combined preparation for simultaneous, separate or sequential use in the treatment or prevention of pain or nociception.

2. A product according to claim 1 wherein the NR2B antagonist is eliprodil or ifenprodil.

3. A product according to claim 1 or 2 wherein the COX-2 inhibitor is celecoxib, or rofecoxib

4. A pharmaceutical composition comprising a selective NMDA NR2B antagonist and a COX-2 inhibitor; together with at least one pharmaceutically acceptable carrier or excipient.

5. The use of a selective NMDA NR2B antagonist and a COX-2 inhibitor for the manufacture of a medicament for the treatment of pain or nociception.

6. A process for the preparation of a pharmaceutical composition comprising a selective NMDA NR2B antagonist and a COX-2 inhibitor, which process comprises bringing a selective NMDA NR2B antagonist and a COX-2 inhibitor into association with a pharmaceutially acceptable carrier or excipient.

## Patentansprüche

1. Ein Produkt, das einen selektiven NMDA-NR2B-Antagonisten und einen COX-2-Inhibitor als ein Kombinationspräparat zur gleichzeitigen, getrennten oder sequentiellen Verwendung bei der Behandlung oder Prävention von Schmerz oder Nozizeption enthält.

2. Ein Produkt gemäß Anspruch 1, wobei der NR2B-Antagonist Eliprodil oder Ifenprodil ist.

3. Ein Produkt gemäß Anspruch 1 oder 2, wobei der COX-2-Inhibitor Celecoxib oder Rofecoxib ist.

4. Eine pharmazeutische Zusammensetzung, die einen selektiven NMDA-NR2B-Antagonisten und einen COX-2-Inhibitor zusammen mit wenigstens einem pharmazeutisch annehmbaren Träger oder Hilfsstoff enthält.

5. Die Verwendung eines selektiven NMDA-NR2B-Antagonisten und eines COX-2-Inhibitors zur Herstellung eines Medikaments zur Behandlung von Schmerz oder Nozizeption.

6. Ein Verfahren zur Herstellung einer pharmazeutischen Zusammensetzung, die einen selektiven NMDA-NR2B-Antagonisten und einen COX-2-Inhibitor enthält, wobei das Verfahren das Zusammenbringen eines selektiven NMDA-NR2B-Antagonisten und eines COX-2-Inhibitors mit einem pharmazeutisch annehmbaren Träger oder Hilfsstoff umfaßt.

## Revendications

1. Produit comprenant un antagoniste sélectif de NMDA NR2B et un inhibiteur de COX-2 en tant que préparation combinée pour une utilisation simultanée, séparée ou séquentielle dans le traitement ou la prévention de la douleur ou de la nociception.

2. Produit selon la revendication 1 dans lequel l'antagoniste de NR2B est l'éliprodil ou l'ifenprodil.

3. Produit selon la revendication 1 ou 2 dans lequel l'inhibiteur de COX-2 est le célécoxib, ou le rofécoxib.

4. Composition pharmaceutique comprenant un antagoniste sélectif de NMDA NR2B et un inhibiteur de COX-2, conjointement avec au moins un support ou excipient pharmaceutiquement acceptable.

5. Utilisation d'un antagoniste sélectif de NMDA NR2B et d'un inhibiteur de COX-2 pour la fabrication d'un médicament destiné au traitement de la douleur ou de la nociception.

6. Procédé de préparation d'une composition pharmaceutique comprenant un antagoniste sélectif de NMDA NR2B et un inhibiteur de COX-2, lequel procédé comprend l'apport d'un antagoniste sélectif de NMDA NR2B et un inhibiteur de COX-2 en association avec un support ou excipient pharmaceutiquement acceptable.
